# EUROPEAN PATENT SPECIFICATION

(11) **EP 1 995 230 B1**
(45) Date of publication and mention of the grant of the patent: **10.04.2013**
(21) Application number: 07714254.5
(22) Date of filing: 15.02.2007
(51) Int. Cl.: C07C 37/84, C07C 39/16

(54) **PROCESS FOR PRODUCING BISPHENOL A**
VERFAHREN ZUR HERSTELLUNG VON BISPHENOL A
PROCÉDÉ DE SYNTHÈSE DU BISPHÉNOL A

(30) Priority: 16.03.2006 JP 2006073385
(43) Date of publication of application: 26.11.2008
(73) Proprietor: Idemitsu Kosan Co., Ltd., Chiyoda-ku Tokyo 100-8321 (JP); Tsukishima Kikai Co., Ltd., Tokyo 104-0051 (JP)
(72) Inventor: YOSHITOMI, Kazuyuki, Ichihara-shi, Chiba 2990193 (JP); KODAMA, Masahiro, Ichihara-shi, Chiba 299-0193 (JP); MASUDA, Shuichi, Ichihara-shi, Chiba 299-0193 (JP); IWASAKI, Shuji, Ichikawa-shi, Chiba 2720127 (JP); HOMMA, Tomoki, Chuo-ku, Tokyo 1040051 (JP); SUDA, Hideki, Chuo-ku, Tokyo 1040051 (JP)
(74) Representative: Vossius & Partner
(86) International application number: PCT/JP2007/052724
(87) International publication number: WO 2007/108259

(56) References cited:
- EP-A1- 1 541 542
- WO-A1-2004/020377
- JP-A- 07 025 798
- JP-A- 07 047 209
- JP-A- 2004 137 197
- JP-A- 2004 359 594

## Description

### [Technical Field]

The present invention relates to a process for producing bisphenol A using a horizontal belt filter equipped with a filter cloth having a long operating life and a capability of exerting a stable filtration performance in solid/liquid separation of slurry containing an adduct of bisphenol A and phenol.

### [Background Art]

Bisphenol A, used as a raw material for polycarbonate that has a growing demand as an engineering plastic in recent years, is produced by reacting acetone and an excess amount of phenol in the presence of an acid catalyst.
In a conventional method for recovering bisphenol A from the resulting reaction mixture, an adduct of bisphenol A and phenol is deposited by concentrating and cooling after acetone and water are removed from the reaction mixture, and then the reaction mixture is subjected to solid/liquid separation. A solid/liquid separation method using a drum filter, a centrifugal separator, a horizontal belt filter, and the like has been known.
For example, in Patent Documents 1 and 2, a solid/liquid separation method using a horizontal belt filter is described. The horizontal belt filter is equipped with a filter cloth and is operated at a speed of 1 to 5 m/min. The horizontal belt filter is constructed as slurry is supplied onto the filter cloth, filtered, and washed; the adduct of bisphenol A and phenol is taken out as a solid cake; on the other hand, the filtrate or a washing liquid used to wash the adduct is discharged, through a vacuum tray that moves back and forth several times per minutes, from a hose connected to the vacuum tray.
The filter cloth loaded with a thick solid cake is constantly contacted to the vacuum tray, so that the filter cloth is abraded by friction with the vacuum tray upon each back and forth motion of the vacuum tray.
In addition, the horizontal belt filter body is exposed to a tough condition of heated acidic phenol gas that is highly corrosive to metal or various fibrous materials.
Patent Document 3 discloses a process for producing bisphenol A which comprises crystallizing an adduct of bisphenol A with phenol from a solution of bisphenol A in phenol to form a slurry, said bisphenol A being produced by reacting phenol and acetone in the presence of an acid catalyst, subjecting the resultant slurry to a solid-liquid separation treatment, and thereafter removing the phenol from solid components, characterized by pouring onto a filter, a slurry solution of bisphenol A in phenol, said slurry solution containing in a crystalline state, an adduct of bisphenol A with phenol having an average particle size in the range of 0.05 to 1 mm, and filtering said slurry solution under reduced pressure in an atmosphere of an inert gas stream at 30 to 80 °C containing oxygen in a concentration of at most 5,000 ppm by volume to constitute an adduct layer of bisphenol A with phenol.>

Patent Document 1: Japanese Patent Laid-Open Publication No. H7-47209,
Patent Document 2: Japanese Patent Laid-Open Publication No. 2004-137197,
Patent Document 3: EP 1 541 542 A.

### [Disclosure of the Invention]

### [Problems to be Solved by the Invention]

The present invention has been made to address the problems under the aforementioned circumstances. It is an object of the present invention to provide a process for producing bisphenol A using a horizontal belt filter equipped with a filter cloth that has a long operating life and a capability of exerting a stable filtration performance in a solid/liquid separation process of slurry containing an adduct of bisphenol A and phenol.

### [Means for Solving the Problems]

The present inventors have made intensive studies to achieve the aforementioned object. As a result, the present inventors have found that the object can be achieved by the process as defined below by using a horizontal belt filter equipped with a filter cloth that is woven out of yarns having uniform diameters and has an air permeability of 50 to 100 ml/cm² ·sec. Based on this finding, the present invention has been accomplished.

Namely, the present invention provides:
(1) A process for producing bisphenol A
   , comprising:
   crystallizing an adduct of bisphenol A and phenol out of a phenol solution of bisphenol A obtained by reacting phenol and acetone in the presence of an acid catalyst, and solid / liquid separating slurry obtained in the crystallizing step by using horizontal belt filter cloth which is woven out of a yarn having a uniform diameter and having an air permeability of 50 to 100 ml/cm²·sec measured in accordance with JIS L-1096
   wherein the filter cloth is woven out of a yarn having a diameter deviation of ± 0.1 mm or less; and
   wherein the filter cloth is woven out of a warp yarn with a diameter of 0.20 to 0.40 mm and a weft yarn with a diameter of 0.6 to 0.8 m.
(2) The process for producing bisphenol A as described in (1),
   wherein the filter cloth is a herringbone weave cloth woven out of two warp yarns and two weft yarns;
(3) The process for producing bisphenol A as described in (1) or (2), wherein the yarn is made of a thermoplastic resin fiber or a natural fiber;
(4) The process for producing bisphenol A as described in (3), wherein the yarn made of a thermoplastic resin fiber is a yarn made of a polypropylene, polyester, nylon, or polytetrafluoroethylene fiber;
(5) The process for producing bisphenol A as described in (3), wherein the yarn made of a natural fiber is a yarn made of cotton or hemp;
(6) The process for producing bisphenol A as described in any one of (1) to (5), wherein the filter cloth has a lacing portion at which the filter cloth is bonded with a stainless steel hook or nickel alloy hook and a stainless steel wire, and a hot-melt adhesive of maleic acid modified polypropylene is applied to the filter cloth portion in which the hook is punched;
(7) The process for producing bisphenol A as described in (8), wherein a yarn made of a thermoplastic resin fiber is interposed in the lacing portion of the filter cloth; and
(8) The process for producing bisphenol A as described in (7), wherein the yarn that is made of a thermoplastic resin fiber and is interposed in the lacing portion of the filter cloth is a yarn made of a polypropylene fiber.

### [Brief Description of Drawings]

FIG. 1 is a schematic view of a solid/liquid separation unit using a horizontal belt filter according to the present invention.
FIG. 2 is a view exemplifying a lacing portion of a filter cloth equipped in a horizontal belt filter according to the present invention.

### [Explanation of Reference Numerals]

1: Phenol adduct crystals
2: Filter cloth
3: Vacuum tray
4: Liquid discharge line
5: Hook
6: Stainless steel wire
7: Thermoplastic resin fiber yarn
8: Maleic acid modified polypropylene holt-melt adhesive

### [Best Mode for Carrying Out the Invention]

The process for producing bisphenol A according to the present invention is characterized by using a horizontal belt filter equipped with a filter cloth that has a long operating life and a capability of exerting a stable filtration performance in solid/liquid separation of slurry that contains an adduct of bisphenol A and phenol.

In the process for producing bisphenol A according to the present invention, bisphenol A is produced through the following steps: (A) a step of reacting phenol and acetone; (B) a step of removing low boiling-point substances including by-produced water and unreacted raw material; (C) a step of concentration of bisphenol A; (D) a step of crystallization and solid/liquid separation; (D') a step of dissolving, crystallizing and solid/liquid separating phenol adduct crystals; (E) a step of heating and melting; (F) a step of removing phenol from bisphenol A; and (G) a step of granulating.
In the present invention, a horizontal belt filter is used for the solid/liquid separation in the step (D) and/or the step (D'). The horizontal belt filter is equipped with a filter cloth that has a long operating life and a stable filtration performance, thereby providing a process for producing stably bisphenol A.

Each step in the process for producing bisphenol A is explained below.

### (A) Reaction step

In this reaction step, an excess amount of phenol is condensed with acetone in the presence of an acid catalyst to form bisphenol A. As the acid catalyst, may be used an acid ion-exchange resin. The acid ion-exchange resin is not particularly limited, but a conventional catalyst used for the production of bisphenol A may be used. In particular, considering catalytic activity, a sulfonic acid cation-exchange resin may be suitably used. The sulfonic acid cation-exchange resin is not particularly limited as long as it is a strongly acidic cation-exchange resin having a sulfonic acid group. Examples of the sulfonic acid cation-exchange resin may include sulfonated styrene/divinylbenzene copolymer, sulfonated cross-linked styrene polymer, phenol formaldehyde/sulfonic acid resin, benzene formaldehyde/sulfonic acid resin, and the like. These may be used solely or two or more kinds in combination.

In the present step, along with the acid ion-exchange resin, mercaptans serving as an auxiliary catalyst are usually used in combination. The mercaptans are a compound having a free SH group in the molecule. The mercaptans may include alkyl mercaptans or alkyl mercaptans having at least one kind of substitution group such as carboxyl group, amino group, and hydroxyl group, including, for example, mercapto carboxylic acids, aminoalkane thiols, mercapto alcohols, or the like. Examples of the mercaptans may include an alkyl mercaptan such as methyl mercaptan, ethyl mercaptan, n-butyl mercaptan, and n-octyl mercaptan; a thiocarboxylic acid such as thio glycolic acid and β-mercapto propionic acid; an aminoalkane thiol such as 2-aminoethane thiol; a mercapto alcohol such as mercapto ethanol; and the like. Among these, alkyl mercaptan is particularly suitable considering the effect as an auxiliary catalyst. Further, these mercaptans may be used solely or two or more kinds in combination. These mercaptans may be fixed on the aforementioned acid ion-exchange resin so as to serve as an auxiliary catalyst.
The used amount of the mercaptans is selected in the range of generally from 0.1 to 20 mol% and preferably from 1 to 10 mol% in terms of the acetone as a raw material.

The ratio of phenol to acetone used is not particularly limited, but it is desirable that the amount of unreacted acetone is limited as small as possible considering easiness of purification of the produced bisphenol A or from the economical viewpoint. Therefore, it is advantageous that phenol is used in excess over the stoichiometric amount. Usually, phenol is used in an amount of 3 to 30 mol per one mole of acetone, and preferably 5 to 15 mol. Further, in the bisphenol A production, usually a reaction solvent is not needed except the case where the viscosity of the reaction liquid is too high or the case where the reaction is disturbed by solidification at low temperatures.

The condensation reaction between phenol and acetone may be performed in a batch-wise or a continuous process, but a fixed-bed continuous process is advantageous where acetone and phenol are reacted while supplying continuously phenol, acetone, and mercaptans (in the case where mercaptans are not fixed on an acid ion-exchange resin) to a reactor loaded with acid ion-exchange resin. In this process, the reactor may be one or two or more in series, but industrially it is particularly advantageous that two or more reactor loaded with the acid ion-exchange resin are connected in series and a fixed-bed multi-stage continuous process is applied.

Reaction conditions applied to the fixed-bed continuous process are explained.
Firstly, the molar ratio of acetone/phenol is selected in the range of usually from 1/30 to 1/3 and preferably from 1/15 to 1/5. When the molar ratio is smaller than 1/30, the reaction rate possibly becomes low. At a molar ratio of larger than 1/3, the amount of impurities produced becomes large, thereby the selectivity for bisphenol A is likely to be lowered. On the other hand, in the case where mercaptans are not fixed on the acid ion-exchange resin, the molar ratio of mercaptans/acetone is selected in the range of usually from 0.1/100 to 20/100 and preferably from 1/100 to 10/100. When the molar ratio is smaller than 0.1/100, possibly an effect of enhancing the reaction rate or the selectivity for bisphenol A is not exerted sufficiently. At a molar ratio of larger than 20/100, the amount increased seems not to pay for the increase of the effect.
The reaction temperature is selected in the range of usually from 40 to 150°C and preferably from 60 to 110°C. When the temperature is lower than 40°C, the reaction rate becomes low, and the viscosity of the reaction liquid becomes extremely high and the reaction liquid is possibly solidified in some cases. At a reaction temperature of higher than 150°C, the reaction becomes difficult to control, the selectivity for bisphenol A (p,p'-form) is lowered, and the acid ion-exchange resin serving as a catalyst is possibly decomposed or degraded. Further, the LHSV (Liquid Hourly Space Velocity) of the raw material mixture is selected in the range of usually from 0.2 to 30 hr⁻¹ and preferably from 0.5 to 10 hr⁻¹.

### (B) Step of removing low boiling-point substances

In the step of removing low boiling-point substances, the reaction mixture containing bisphenol A obtained in the reaction step (A) is subjected to a treatment of removing low boiling-point substance under the condition where the reaction mixture is substantially free of the acid ion-exchange resin, that is, after the catalyst is removed by filtration and the like in the case of the batch-wise process, or as it is in the case of the fixed bed continuous process.
In this step, generally, firstly low boiling-point substances such as unreacted acetone, by-produced water, and alkyl mercaptan are removed by vacuum distillation using a distillation column. The vacuum distillation is performed usually under a pressure of about 6.5 to 80 kPa and at a temperature of about 70 to 180°C. On this occasion, unreacted phenol is azeotroped and a part of it is discharged out of the reaction system along with the low boiling-point substances from the head of the distillation column. In this vacuum distillation, it is desirable that the temperature of a heat source used is kept at 190°C or lower so as to prevent thermal decomposition of bisphenol A. Further, the material of the apparatuses or instruments used here is selected usually from SUS304, SUS316, and SUS316L.

### (C) Concentration step

In a reactor bottom liquid that is given by removing the low boiling-point substances from the reaction mixture, bisphenol A, phenol, and others are contained, so that phenol is removed by vacuum distillation to concentrate bisphenol A.
The conditions for the concentration are not particularly limited, but the concentration is performed usually at a temperature of about 100 to 170°C under a pressure of about 5 to 70 kPa. When the temperature is lower than 100°C, high vacuum is needed. When the temperature is higher than 170°C, an extra operation of removing heat is needed in the following crystallization step. The bisphenol A concentration in the resulting concentrated liquid is in the range of preferably from 20 to 50% by mass and more preferably from 20 to 40% by mass. When the concentration is lower than 20% by mass, the recovery of bisphenol A is low. At a concentration higher than 50% by mass, possibly the slurry obtained after crystallization becomes not easy to convey.

### (D) Crystallization and solid/liquid separation step

In the crystallization and solid/liquid separation step, a 1:1 adduct (hereinafter, also referred to as phenol adduct crystals) of bisphenol A and phenol is crystallized and separated from the concentrated liquid obtained in the step (C). In this step, firstly, the concentrated liquid is cooled to about 40 to 70°C so as to crystallize the phenol adduct and to obtain slurry. The concentrated liquid can be cooled with an external heat-exchanger or by using the method of vacuum-cooling crystallization in which water is added to the concentrated liquid so as to cool it with the latent heat of water vaporization under vacuum. In the vacuum-cooling crystallization, after water is added in an amount of about 3 to 20% by mass to the concentrated liquid, usually, the liquid is crystallized at a temperature of about 40 to 70°C under a pressure of about 4 to 16 kPa. When the amount of water added is less than 3% by mass, sufficient heat removal effect is not expected. When the amount goes over 20% by mass, possibly the solubility of bisphenol A becomes increased.

In the crystallization, when the crystallization temperature is lower than 40°C, possibly the viscosity of the crystallizing liquid becomes increased or the liquid is solidified. At a temperature of higher than 70°C, the solubility of bisphenol A possibly becomes increased.
After that, the slurry that contains the crystallized phenol adduct crystals is introduced onto a horizontal belt filter in a heated inert gas stream under vacuum so as to form a phenol adduct crystal layer on the filter.
By using the horizontal belt filter, filtration can be carried out continuously. In addition, filtration can be advantageously carried out without a large gravitational force.
On this occasion, the average particle size of the phenol adduct crystals is preferably about 0.05 to 1 mm. When the average particle size is less than 0.05 mm, separation between the phenol adduct crystals and mother liquid becomes difficult, thereby bringing about clogging in the filter and lowering in filtration efficiency. When the average particle size is larger than 1 mm, the mother liquid containing impurities is incorporated into the crystals. Thereby, the purity of the phenol adduct crystals is lowered.
By separating the mother liquid contained in the phenol adduct crystal layer through the horizontal belt filter, the liquid content in the phenol adduct crystal layer is reduced to 30% by mass or less and preferably 25% by mass or less. In order that the layer may fall off by own weight in a form of a solid cake from the horizontal belt filter, the liquid content in the phenol adduct crystal layer is preferably 30% by mass or less. The lower the liquid content of the adduct deposited on the filter, the lighter the burden in the following steps.

The mother liquid and the phenol adduct crystals are separated efficiently by filtration under vacuum, so that the liquid content in the phenol adduct crystal layer can be lowered. However, too much vacuum sometimes brings about lowering in the mother liquid temperature and leads to generation of fine crystals, thereby possibly accelerating trouble such as clogging of the filter cloth. A preferable degree of vacuum is about 60 to 95 kPa.
The liquid content in the phenol adduct crystal layer is influenced by the running speed (belt speed) of the horizontal belt filter, so that the liquid content in the phenol adduct crystal layer can be reduced to 30% by mass or less by regulating the degree of vacuum and belt speed of the horizontal belt filter.
As long as the phenol adduct crystals having a liquid content of 30% by mass or less are deposited on the horizontal belt filter, the thickness of the phenol adduct crystal layer is not limited. However, when the layer becomes too thick, the increased mass per unit area becomes a burden to the apparatus. Note that, before the phenol adduct crystal layer falls off by its own weight from the horizontal belt filter, the phenol adduct crystal layer is desirably washed by spraying a washing liquid thereon so as to remove impurities and small amount of the acid catalyst from the phenol adduct crystal layer to a maximum extent. The washing liquid may include phenol, water, a mixed liquid of water and phenol, or a liquid dissolving bisphenol A therein.
The thickness of the phenol adduct crystal layer and the time of vacuum suction can be regulated by the belt speed. As the belt speed goes slower, the thickness of the phenol adduct crystal layer becomes thicker, while the layer can be treated under vacuum over a longer period of time. In order to keep the slurry that contains the phenol adduct crystals crystallized upon filtration in a slurry state, the temperature is required to be kept at 80°C or lower. In addition to that, the mother liquid or the washing liquid is possibly solidified, so that it is essential that the temperature of the atmosphere around the filter be kept at about 30 to 80°C and preferably about 45 to 60°C.

All or a part of the mother liquid separated may be returned to the reactor, or all or a part of the mother liquid separated may be isomerized and returned to the crystallization step. In order to obtain a high purity product, it is effective that this crystallization and solid/liquid separation step (D) are repeated two or more times. That is, preferably, the phenol adduct crystals obtained by solid/liquid separation after crystallization are moved to the step (E) of heating and melting, after the following step (D') of dissolving, crystallizing, and solid/liquid separating the phenol adduct crystals is repeated at least one time.

### (D') Step of dissolving, crystallizing and solid/liquid separating phenol adduct crystals

In this step, the phenol adduct crystals crystallized and separated in the step (D) are dissolved in a phenol-containing solution. The phenol-containing solution used in this step is not particularly limited. Examples of the solution may include the phenol recovered in the concentration step (C); the washing liquid for the phenol adduct crystals formed in the crystallization and solid/liquid separation step (D); the mother liquid or the washing liquid for the phenol adduct crystals that is obtained after solid/liquid separation of the crystallized phenol adduct crystals in this step (D') or the following steps; and the like.
In this step, the phenol-containing solution is added to the phenol adduct crystals obtained in the step (D) and heated at about 80 to 110°C to dissolve the phenol adduct crystals by heating, so that a bisphenol A containing solution having a bisphenol A concentration adequate for crystallization is prepared. Thus prepared bisphenol A containing solution is relatively easy to handle because it has a low viscosity at relatively low temperatures, and is suitable for filtration with a horizontal belt filter.
In this way, phenol adduct crystals are crystallized and solid/liquid separated from the bisphenol A containing solution; further the phenol adduct crystals are dissolved in the phenol-containing solution; and then the crystallization and solid/liquid separation performed in the step (D) is repeated at least one time.
The mother liquid or the washing liquid for the phenol adduct crystals that is solid/liquid separated in this step may be used again as an adduct washing liquid for the solid/liquid separation unit in the step (D) or may be used again in place of water when vacuum-vaporization crystallization is performed in the step (D).

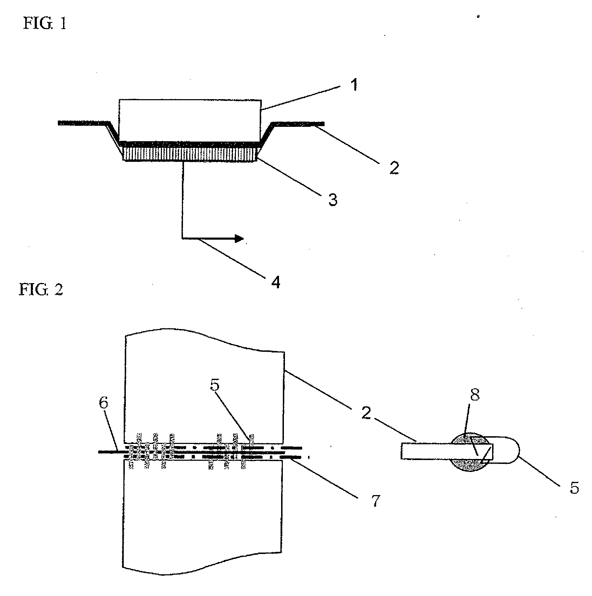

The horizontal belt filter used in the present invention is designed in an endless configuration. A filter cloth is supported by stretching it with several rollers and the filter cloth runs over a vacuum tray. The slurry is supplied to the one end of the filter cloth over the vacuum tray so as to filter off the phenol adduct crystals while the resulting cake of the phenol adduct crystals is washed. The cake is removed from the filter by its own weight. As the filter cloth of the horizontal belt filter, is used a porous cloth woven out of a yarn as defined above, preferably a thermoplastic resin fiber yarn, for example, a yarn of polypropylene, polyester, nylon, or polytetrafluoroethylene fibers, or a natural fiber yarn, for example, a yarn made of cotton, hemp, or the like. Particularly preferably is used a filter cloth made of a yarn of polypropylene fibers that have an excellent resistance against phenol and are low cost and versatile.
The vacuum tray moves forward (the same direction as the filter cloth runs) for about 15 seconds and moves backward (in the opposite direction to the filter cloth runs) for about 5 seconds, repeatedly. Because a cake of the phenol adduct crystals several 10s mm in thick is loaded on the filter cloth that constantly moves with a drum roller installed in the horizontal belt filter, the filter cloth and vacuum tray constantly repeatedly receive friction against each other (see FIG. 1). Due to this, irregularity in the diameter of a yarn out of which the filter cloth is woven brings about local expansion, abrasion by friction, and worn-out. Ultimately the filter cloth is fractured, thereby causing a fatal problem of stopping the operation of the bisphenol A production plant. On the other hand, when a filter cloth that is woven densely is selected in order to avoid the abrasion, the air permeability becomes insufficient. The problem is that an expected filtration performance is hardly exerted.

The filter cloth used in the present invention has a long operating life and a capability of exerting a stable filtration performance. Specifically, the filter cloth is woven out of a yarn as defined above, preferably a thermoplastic resin fiber yarn having a uniform diameter, for example, a yarn made of polypropylene, polyester, nylon or polytetrafluoroethylene fibers, or a natural fiber yarn, for example, a yarn made of cotton, hemp or the like, preferably a yarn made of polypropylene fibers. The fiber cloth has an air permeability of 50 to 100 ml/cm²·sec (in accordance with JIS L-1096) and preferably 65 to 75 ml/cm²·sec. A filter cloth having an air permeability of less than 50 ml/cm²·sec has a large resistance against gas permeation, thereby lowering the filtration performance because the amount of gas permeated becomes insufficient. On the other hand, a filter cloth having an air permeability of higher than 100 ml/cm²·sec brings about a difficulty in continuous operation because the crystals passed through the filter cloth gradually clog the vacuum tray and prevent the flow of the filtrate and gas. It is required to dissolve and remove the crystals with solvent such as phenol while the production is intermitted and the belt filter is stopped.
In addition, a filter cloth woven out of a yarn having a uniform diameter with a deviation of ± 0.1 mm or less is used. Further, a herringbone weave filter cloth woven out of two warp yarns and two weft yarns is preferable. The herringbone weave filter cloth woven out of two warp yarns and two weft yarns runs stably in the horizontal belt filter (the cloth is crease-resistant, and snaky traveling is inhibited) and has a capability of keeping an adequate strength as a filter cloth.

The horizontal belt filter used in the bisphenol A production is a large size machine. A filter cloth loaded in this large-size machine is required to have a wide width of about 3 to 5 m, a long length of about 20 to 40 m, the aforementioned air permeability ensuring a high filtration performance, and a high durability.
A filter cloth that satisfies the above requirements is woven out of a warp yarn having a diameter of 0.20 to 0.40 mm and a weft yarn having a diameter of 0.6 to 0.8 mm. A filter cloth woven out of the yarns having the above diameters prevents wrinkles, keeps an adequate strength as a filter cloth, has an air permeability required as described above, and hardly brings about a problem in separability against the phenol adduct crystals. Here, the yarn diameter is measured with a dial gauge.
A filter cloth woven out of yarns having uniform diameters is loaded in a horizontal belt filter, and then the both ends of the filter cloth are required to be bonded. The filter cloth is bonded with a plastic hook, a nickel-alloy hook, a stainless steel hook, a fastener, and the like.
In the case where the filter cloth is bonded with the stainless steel hook or the nickel-alloy hook, hooks bonded to the ends of the filter cloth are intersected to each other and a stainless steel wire is passed through the intersected portion so as to bond the ends of the filter cloth together (hereinafter, this bonded portion is also called as a lacing portion). In this case, these hooks are pulled with a large force, they are gradually abraded, and ultimately they are possibly broken and separated from each other. In order to address this problem, a hot-melt adhesive of maleic acid modified polypropylene is preferably applied to the filter cloth portions in which the hooks are punched. By this application, the operating life of the filter cloth lacing portion, that is, the operating life of the filter cloth can be extended.
In some cases, the hooks are dropped off, the wire is then dropped off, and the cloth are subsequently disconnected, ultimately the horizontal belt filter stops operation. This causes a significant problem of shutdown of the bisphenol A production plant.
Note that, specifically, the stainless steel hook may be made of SUS316L, SUS316, and the like. The nickel-alloy hook may be made of Hastelloy C (registered trade mark), and the like (see FIG. 2).

At the lacing portion of the filter cloth, a spacing of about 3 to 5 mm is developed. The cake of the phenol adduct crystals is not loaded on the spacing, but only gas passes through it. Namely, the gas passing through the phenol adduct crystal layer and properly contributing to filtration leaks out from this spacing, thereby lowering the filtration performance.
Due to this, in order to enhance the filtration performance of the filter cloth, it is preferred that a yarn made of thermoplastic resin fibers, preferably polypropylene fibers, is interposed into the spacing of the lacing portion of the filter cloth and embedded the spacing.

### (E) Heating and melting step

In the heating and melting step, the phenol adduct crystals crystallized and separated in the step (D) or step (D') are heated and melted. In this step, the phenol adduct crystals are heated and melted into a liquid mixture at about 100 to 160°C.

### (F) Phenol removing step

In the phenol removing step, phenol is distilled out by vacuum distillation from the phenol adduct crystals that are heated and melted in the step (E), so that a bisphenol A melt is recovered. The vacuum distillation is usually performed under a pressure of about 1.3 to 13.3 kPa and at a temperature of about 150 to 190°C. The remaining phenol can be further removed by steam stripping.

### (G) Granulation step

In the granulation step, the bisphenol A melt obtained in the step (F) is transformed into liquid drops with a granulation apparatus such as a spray dryer, and then cooled and solidified into a product. The liquid drops are formed by spraying, scattering, or the other method and cooled with nitrogen gas, air, or the like.

### [Examples]

Hereinafter, the present invention will be described in detail with reference to the following examples, but it should be construed that the present invention is in no way limited to those examples.

### Example 1

Through a fixed-bed reactor loaded with a catalyst of a cation-exchange resin ("DIAION SK104H" (registered trade mark), manufactured by Mitsubishi Chemical Corporation) whose sulfonic acid groups were partly neutralized by 20 mol% with 2-mercapto ethylamine, phenol and acetone (phenol: 50 t/hr, acetone: 2t/hr) were allowed to flow continuously at an LHSV of 3 hr⁻¹ and react at 75°C. The resulting reaction mixture was introduced into a vacuum distillation column. After acetone, water and others were removed by vacuum distillation at a column bottom temperature of 170°C under a pressure of 67 kPa, phenol was further distilled out by vacuum distillation at a temperature of 130°C under a pressure of 14 kPa until bisphenol A was concentrated to 40% by mass. After water was added at a rate of 4 t/hr, the reaction mixture was kept cooled at 50°C under vacuum so as to crystallize adduct crystals of bisphenol A and phenol. A slurry liquid having a slurry concentration of 35% by mass with a mother liquid having a 2, 4-isomer concentration of 6% by mass was obtained at a rate of 40 t/hr.
The resulting slurry was poured onto a horizontal belt filter in a casing in a 70°C nitrogen gas with a polypropylene filter cloth running at a speed of 2 m/min so as to be separated the slurry into phenol adduct crystals and mother liquid under a pressure of 60 kPa with 5 seconds. The phenol adduct crystals separated from the mother liquid formed an 80 mm thick cake layer. After the mother liquid still remained in the cake layer was removed, the cake layer was washed with phenol by repeating 10 second washing two times (washing ratio was 0.75), filtrated and dried. The cake layer was allowed to drop off by its own weight at the end of the horizontal belt filter, so that wet phenol adduct crystals were obtained.
The specifications of the polypropylene filter cloth used are as follows.
The air permeability was 70 ml/cm² sec. The filter cloth was a herringbone weave cloth 3.5 m wide, 30 m long woven out of two warp yarns that met the 0.28 mm (deviation of yarn diameter was ± 0.05 mm) production standard for warp yarns and two weft yarns that met the 0.7 mm (deviation of yarn diameter was ± 0.05 mm) production standard for weft yarns. Stainless steel (SUS316L) hooks were punched in a lacing portion of the filter cloth, which were then bonded with a stainless steel (SUS316) wire. A hot-melt adhesive of maleic acid modified polypropylene was applied to the portion where the hooks were punched. To the lacing portion of the filter cloth, four yarns of polypropylene were interposed so as to embed the spacing.

### Comparative Example 1

Except that a filter cloth having an air permeability of 40 ml/cm² sec was used, the same specification of the filter cloth and the same operation conditions as in Example 1 were applied.

### Comparative Example 2

Comparative Example 2 was carried out similarly to Example 1, except that a filter cloth having an air permeability of 150 ml/cm²·sec was used.

### Comparative Example 3

Comparative Example 3 was carried out similarly to Example 1, except that a filter cloth woven out of yarns having non-uniform diameters with the 0.28 mm (deviation of yarn diameter was ± 0.2 mm) production standard for warp yarns and the 0.7 mm (deviation of yarn diameter was ± 0.4 mm) production standard for weft yarns was used.

### Example 2

Except that, in the specification of the filter cloth, the hot-melt adhesive of maleic acid modified polypropylene was not applied to the filter cloth portion where the hooks were punched, the same specification of the filter cloth and the same operation conditions as in Example 1 were applied.

### Example 3

Except that the yarn of polypropylene fibers was not interposed in the lacing portion of the filter cloth in the specification of the filter cloth, the same specification of the filter cloth and the same operation conditions as in Example 1 were applied.

The specifications of the filter cloths used in Examples and Comparative Examples, filtration performance, continuous operation performance over one year, worn-out or not of the filter cloths, dropping off or not of the hooks, falling off or not of the cake, abrasion property of filter cloths, and others are shown together in Table 1.

**[Table 1]**

| | Air permeability (ml/cm²·s) | yarn diameter(mm) | | Yarn interposing at lacing portion | Use of maleic acid modified hot melt resin | Liquid content in adduct (% by mass) | 2,4-isomer concentration (% by mass) | Continuous operability over one year | Filter cloth worn out | Hook dropping off | Cake falling off | Filter cloth abrasion |
|---|---|---|---|---|---|---|---|---|---|---|---|---|
| | | Warp yarn (yarn diameter deviation) | Weft yarn (yarn diameter deviation) | | | | | | | | | |
| Example 1 | 70 | 0.28 (± 0.05) | 0.7 (±0.05) | Yes | Yes | 25 | 0.10 | Good | No | No | No | Uniform |
| Comparative Example 1 | 40 | 0.28 (± 0.05) | 0.7 (±0.05) | Yes | Yes | 35 | 0.17 | Good | No | No | No | Uniform |
| Comparative Example 2 | 150 | 0.28 (± 0.05) | 0.7 (±0_{.}05) | Yes | Yes | 24 | 0.09 | No good (only 2 months) | No | No | No | Uniform |
| Comparative Example 3 | 70 | 0.28 (± 0.2) | 0.7 (± 0.4) | Yes | Yes | 25 | 0.10 | No good | Yes | No | No | Non-uniform |
| Example 2 | 70 | 0.28 (± 0.05) | 0.7 (±0.05) | Yes | No | 26 | 0.11 | Good | No | Yes | No | Uniform |
| Example 3 | 70 | 0.28 (± 0.05) | 0.7 (±0.05) | No | Yes | 27 | 0.12 | Good | No | No | No | Uniform |

As is clear from Table 1, in Example 1, the following filtration performance was attained: the liquid content in the wet phenol adduct crystals was 25% by mass; the 2,4-isomer concentration in the obtained wet phenol adduct crystals was 0.10% by mass; and the operation was allowed to continue over one year. The filter cloth was not broken over one year and was abraded uniformly. No stainless steel hooks dropped off. No cake was found to fall off from the lacing portion of the filter cloth.
In Example 2, in which no hot-melt adhesive of maleic acid modified polypropylene was applied to the portion of the filter cloth where hooks were punched, the following filtration performance was attained: the liquid content in the wet phenol adduct crystals was 26% by mass; the 2,4-isomer concentration in the obtained wet phenol adduct crystals was 0.11% by mass; and the operation was allowed to continue over one year. The filter cloth was not broken over one year and was abraded uniformly. However, a part of the stainless steel hooks at the lacing portion were found to drop off when the operation was stopped after one year.
In Example 3, in which no yarn of polypropylene fibers was interposed in the lacing portion of the filter cloth, the following filtration performance was attained: the liquid content in the wet phenol adduct crystals was 27% by mass; the 2, 4-isomer concentration in the wet phenol adduct crystals was 0.12% by mass; and the operation was allowed to continue over one year. The filter cloth was not broken over one year and was abraded uniformly. No stainless steel hooks were found to drop off.

In Comparative Example 1, in which a filter cloth with an air permeability of 40 ml/cm²·sec was used, due to a large resistance of the filter cloth and the resulting deficiency in the amount of gas permeated, the filtration performance was as follows: the liquid content in the wet phenol adduct crystals was 35% by mass, which was far from 25% by mass of filtration performance attained in Example 1; and the 2, 4-isomer concentration in the obtained wet phenol adduct crystals was 0.17% by mass, which was larger than that of Example 1, incredibly by 1.7 times.
In Comparative Example 2, in which a filter cloth with an air permeability of 150 ml/cm²·sec was used, the filtration performance immediately after the operation was started was as follows: the liquid content in the wet phenol adduct crystals was 24% by mass; and the 2, 4-isomer concentration in the wet phenol adduct crystals was 0.09% by mass. However, the air permeability of the filter cloth was as large as 150 ml/cm² sec, so that the crystals were passed through the mesh of the filter cloth and gradually clogged the vacuum tray. Eventually, the amount of gas passed through the horizontal belt filter was lowered and the filtration performance degraded. Due to this, the plant was stopped for 2 days, once every two months, and a troublesome operation of washing the vacuum tray with phenol was carried out so as to dissolve and remove the crystals deposited on the vacuum tray. In this way, continuous operation over one year was not attained. The operation continued for only two months. In the case where the filter cloth was used intermittently, the filter cloth was abraded uniformly even after one year operation and the stainless steel hooks were not dropped off. However, the operation was not allowed to continue for one year because of the reasons described above.
In Comparative Example 3, in which a filter cloth woven out of a yarn having a non-uniform diameter was used, a large worn-out was developed in the filter cloth after 6 month from the start of the operation. The operation was found to be difficult to continue, so that the bisphenol A production plant was stopped and that the filter cloth was replaced. By inspecting the abrasion of the filter cloth, the yarn diameter was found to be non-uniform, that is, the diameter of a part of the weft yarn that wove the filter cloth was extremely larger than the diameter of the weft yarn around the part of the weft yarn. In this thick portion of yarn, the warp yarn was abraded by local friction, thereby causing a worn-out. The worn-out was developed into a large one in a single burst. A filter cloth with the same specifications was loaded again, and the operation was re-started. After 6 month operation, the plant was stopped to inspect the filter cloth. The similar worn-out was found in the filter cloth.

### [Industrial applicability]

According to the present invention, can be provided a process for producing bisphenol A that is characterized by using a horizontal belt filter equipped with a filter cloth having a long operating life and a capability of exerting stable filtration performance in a solid/liquid separation step of slurry that contains an adduct of bisphenol A and phenol.

## Claims

1. A process for producing bisphenol A, comprising :
crystallizing an adduct of bisphenol A and phenol out of a phenol solution of bisphenol A obtained by reacting phenol and acetone in the presence of an acid catalyst, and
solid / liquid separating slurry obtained in the crystallizing step by using horizontal belt filter cloth which is woven out of a yarn having a uniform diameter and having an air permeability of 50 to 100 ml/cm²·sec measured in accordance with J/S L-1096;
wherein the filter cloth is woven out of a yarn having a diameter deviation of ± 0.1 mm or less; and wherein
the filter cloth is woven out of a warp yarn with a diameter of 0.20 to 0.40 mm and a weft yarn with a diameter of 0.6 to 0.8 mm.

2. The process for producing bisphenol A according to claim 1 , wherein
the filter cloth is a herringbone weave cloth woven out of two warp yarns and two weft yarns.

3. The process for producing bisphenol A according to claims 1 or 2, wherein
the yarn is made of a thermoplastic resin fiber or a natural fiber.

4. The process for producing bisphenol A according to claim 3, wherein
the yarn made of a thermoplastic resin fiber is a yarn made of a polypropylene, polyester, nylon, or polytetrafluoroethylene fiber.

5. The process for producing bisphenol A according to claim 3, wherein
the yarn made of a natural fiber is a yarn made of cotton or hemp.

6. The process for producing bisphenol A according to any one of claims 1 to 5, wherein
the filter cloth has a lacing portion at which the filter cloth is bonded with a stainless steel hook or nickel alloy hook and a stainless steel wire, and a hot-melt adhesive of maleic acid modified polypropylene is applied to the filter cloth portion in which the hook is punched.

7. The process for producing bisphenol A according to claim 6, wherein
a yarn made of a thermoplastic resin fiber is interposed in the lacing portion of the filter cloth.

8. The process for producing bisphenol A according to claim 7, wherein
the yarn that is made of a thermoplastic resin fiber and is interposed in the lacing portion of the filter cloth is a yarn made of a poly-propylene fiber.

## Patentansprüche

1. Ein Verfahren zur Herstellung von Bisphenol A, umfassend:
Kristallisieren eines Addukts von Bisphenol A und Phenol aus einer Phenollösung von Bisphenol A, die durch Umsetzen von Phenol und Aceton in Gegenwart eines sauren Katalysators erhalten wird, und
fest/flüssig-Trennen des Slurry, der in dem Kristallisationsschritt erhalten wird, unter Verwendung eines horizontalen Bandfiltergewebes, welches aus einem Garn gewebt ist, das einen einheitlichen Durchmesser aufweist und eine Luftdurchlässigkeit von 50 bis 100 ml/cm²·sec, gemessen in Übereinstimmung mit JIS L-1096, aufweist;
wobei das Filtergewebe aus einem Garn gewebt ist, das eine Abweichung des Durchmessers von ±0,1 mm oder weniger aufweist; und
wobei das Filtergewebe aus einem Kettgarn mit einem Durchmesser von 0,20 bis 0,40 mm und einem Schussgarn mit einem Durchmesser von 0,6 bis 0,8 mm gewebt ist.

2. Das Verfahren zur Herstellung von Bisphenol A gemäß Anspruch 1, wobei das Filtergewebe ein Fischgrätmustergewebe, das aus zwei Kettgarnen und zwei Schussgarnen gewebt ist, ist.

3. Das Verfahren zur Herstellung von Bisphenol A gemäß Anspruch 1 oder 2, wobei das Garn aus einer thermoplastischen Harzfaser oder einer natürlichen Faser hergestellt ist.

4. Das Verfahren zur Herstellung von Bisphenol A gemäß Anspruch 3, wobei das Garn, das aus einer thermoplastischen Harzfaser hergestellt ist, ein Garn, das aus Polypropylen-, Polyester-, Nylon- oder Polytetrafluorethylenfaser hergestellt ist, ist.

5. Das Verfahren zur Herstellung von Bisphenol A gemäß Anspruch 3, wobei das Garn, das aus einer natürlichen Faser hergestellt ist, ein Garn, das aus Baumwolle oder Hanf hergestellt ist, ist.

6. Das Verfahren zur Herstellung von Bisphenol A gemäß einem der Ansprüche 1 bis 5, wobei das Filtergewebe einen durchbrochen gewebten Bereich (lacing portion) aufweist, in dem das Filtergewebe mit einem Edelstahlhaken oder einem Haken aus Nickellegierung und einem Edelstahldraht verbunden ist, und ein Schmelzklebstoff aus maleinsäuremodifiziertem Polypropylen auf den Bereich des Filtergewebes aufgebracht ist, in welchen der Haken gesteckt wird.

7. Das Verfahren zur Herstellung von Bisphenol A gemäß Anspruch 6, wobei ein Garn, das aus einer thermoplastischen Harzfaser hergestellt ist, in dem durchbrochen gewebten Bereich des Filtergewebes eingefügt ist.

8. Das Verfahren zur Herstellung von Bisphenol A gemäß Anspruch 7, wobei das Garn, das aus einer thermoplastischen Harzfaser hergestellt ist und in dem durchbrochen gewebten Bereich des Filtergewebes eingefügt ist, ein Garn, das aus einer Polypropylenfaser hergestellt ist, ist.

## Revendications

1. Procédé pour produire du bisphénol A comprenant :
la cristallisation d'un produit d'addition de bisphénol A et de phénol dans une solution phénolique de bisphénol A obtenue par réaction de phénol et d'acétone en présence d'un catalyseur acide, et
la séparation solide/liquide d'une suspension obtenue dans l'étape de cristallisation au moyen d'un tissu filtrant à bande horizontale qui est tissé à partir d'un fil ayant un diamètre uniforme et ayant une perméabilité à l'air de 50 à 100 ml/cm².s mesurée selon JIS L-1096 ; où
le tissu filtrant est tissé à partir d'un fil ayant un écart de diamètre de ± 0,1 mm ou moins ; et où
le tissu filtrant est tissé à partir d'un fil de chaîne ayant un diamètre de 0,20 à 0,40 mm et d'un fil de trame ayant un diamètre de 0,6 à 0,8 mm.

2. Procédé pour produire du bisphénol A selon la revendication 1 où
le tissu filtrant est un tissu à armure en chevrons tissé à partir de deux fils de chaîne et de deux fils de trame.

3. Procédé pour produire du bisphénol A selon les revendications 1 ou 2 où
le fil est fait d'une fibre de résine thermoplastique ou d'une fibre naturelle.

4. Procédé pour produire du bisphénol A selon la revendication 3 où
le fil fait d'une fibre de résine thermoplastique est un fil fait d'une fibre de polypropylène, polyester, nylon ou polytétrafluoroéthylène.

5. Procédé pour produire du bisphénol A selon la revendication 3 où
le fil fait d'une fibre naturelle est un fil fait de coton ou de chanvre.

6. Procédé pour produire du bisphénol A selon l'une quelconque des revendications 1 à 5 où
le tissu filtrant a une partie attache au niveau de laquelle le tissu filtrant est lié avec un crochet en acier inoxydable ou un crochet en alliage de nickel et un fil en acier inoxydable, et un adhésif thermofusible de polypropylène modifié avec l'acide maléique est appliqué à la partie du tissu filtrant dans laquelle le crochet est introduit.

7. Procédé pour produire du bisphénol A selon la revendication 6 où
un fil fait d'une fibre de résine thermoplastique est interposé dans la partie attache du tissu filtrant.

8. Procédé pour produire du bisphénol A selon la revendication 7 où
le fil qui est fait d'une fibre de résine thermoplastique et est interposé dans la partie attache du tissu filtrant est un fil fait d'une fibre de polypropylène.
